# EUROPÄISCHE PATENTSCHRIFT

(11) **EP 1 539 822 B1**
(45) Veröffentlichungstag und Bekanntmachung des Hinweises auf die Patenterteilung: **15.08.2007**
(21) Anmeldenummer: 03775148.4
(22) Anmeldetag: 05.09.2003
(51) Int. Cl.: C07K 16/28, G01N 33/577, C12N 5/08, A61K 39/395

(54) **ANTIKÖRPER ZUR ISOLIERUNG UND/ODER IDENTIFIZIERUNG MESENCHYMALER STAMMZELLEN UND VERFAHREN ZUR ISOLIERUNG UND/ODER IDENTIFIZIERUNG MESENCHYMALER STAMMZELLEN**
ANTIBODY FOR ISOLATING AND/OR IDENTIFYING MESENCHYMAL STEM CELLS AND METHOD FOR ISOLATING AND/OR IDENTIFYING MESENCHYMAL STEM CELLS
ANTICORPS PERMETTANT D'ISOLER ET/OU D'IDENTIFIER DES CELLULES SOUCHES MESENCHYMATEUSES ET PROCEDE POUR ISOLER ET/OU IDENTIFIER DES CELLULES SOUCHES MESENCHYMATEUSES

(30) Priorität: 09.09.2002 DE 10242338
(43) Veröffentlichungstag der Anmeldung: 15.06.2005
(73) Patentinhaber: Eberhard-Karls-Universität Tübingen, 72076 Tübingen (DE)
(72) Erfinder: BÜHRING, Hans-Jörg, 72076 Tübingen (DE); LAMMERS, Reiner, 72070 Tübingen (DE); VOGEL, Wichard, 72076 Tübingen (DE)
(74) Vertreter: Otten, Hajo
(86) Internationale Anmeldenummer: PCT/EP2003/009883
(87) Internationale Veröffentlichungsnummer: WO 2004/025293

(56) Entgegenhaltungen:
- WO-A-92/22584
- DATABASE BIOSIS [Online] BIOSCIENCES INFORMATION SERVICE, PHILADELPHIA, PA, US; 16. November 2000 (2000-11-16), GIESERT CHRISTINA ET AL: "The monoclonal antibody W7C5 detects a novel cell surface antigen expressed on CD34+ and CD34-stem cell subsets" XP002277216 Database accession no. PREV200100322401 & BLOOD, Bd. 96, Nr. 11 Part 1, 16. November 2000 (2000-11-16), Seite 663a, 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- YIN AMY H ET AL: "AC133, a novel marker for human hematopoietic stem and progenitor cells" BLOOD, Bd. 90, Nr. 12, 15. Dezember 1997 (1997-12-15), Seiten 5002-5012, XP0002264586 & ISSN: 0006-4971
- VOGEL WICHARD ET AL: "Heterogeneity among human bone marrow-derived mesenchymal stem cells and neural progenitor cells." HAEMATOLOGICA, Bd. 88, Nr. 2, Februar 2003 (2003-02), Seiten 126-133, XP0002262821 & ISSN: 0390-6078
- KUCI SELIM ET AL: "Identification of a novel class of human adherent CD34- stem cells that give rise to SCID-repopulating cells." BLOOD, Bd. 101, Nr. 3, 1. Februar 2003 (2003-02-01), Seiten 869-876, XP002277215 & ISSN: 0006-4971
- M. WÜLLING ET AL.: "The origin of the neoplastic stromal cell in giant cell tumor of bone" HUMAN PATHOLOGY, Bd. 34, 2003, Seiten 983-993,

## Beschreibung

Die vorliegende Erfindung betrifft einen monoklonalen Antikörper zur Isolierung und/oder Identifizierung von mesenchymalen Stammzellen.

Zusätzlich zu Stammzellen für hämatopoetische Zellen finden sich im Knochenmark auch stammzellähnliche Zellen, die Vorläufer von nicht-hämatopoetischen Geweben darstellen. Diese Vorläufer von nicht-hämatopoetischen Geweben wurden ursprünglich u.a. als an Gewebekulturschalen-adhärierende ("plasticadherent") Zellen bezeichnet und in neuerer Zeit als entweder mesenchymale Stammzellen oder Knochenmark-Stroma-Zellen (MSC).

Diese Zellen sind nicht nur wegen ihrer Multipotenz bezüglich der Differenzierung von Interesse, sondern bspw. auch für ihre mögliche Verwendung in der Zell- und Gentherapie.

Das Mesenchym ist das embryonale Bindegewebe, d.h. es ist das multipotente Muttergewebe von allen Formen von Binde- und Stützgeweben, von glatter Muskulatur sowie von der Skelett- und Herzmuskulatur. Mesenchymale Stammzellen können aus dem Knochenmark adulter Menschen gewonnen und isoliert werden. Sie sind multipotent und tragen zur Regeneration von Knochen, Knorpel, Sehnen, Muskeln, Fettgewebe sowie Stroma bei.

Der Antikörper W7C5 ist von Giesert et al., "The monoclonal antibody W7C5 defines a novle surface antigen on hematopoietic stam cells", Annals of the New York Academy of Sciences 938: 175 - 183, als neuer Marker für hämatopoetische Stammzellen beschrieben worden. Gleichzeitig wurde in dieser Veröffentlichung auch gezeigt, daß auch mesenchymale Stammzellen diesen Marker exprimieren. Neuere Studien zeigen, daß dieser Antikörper das CD109-Epitop auf CD34⁺/CD38⁻-Stammzellpopulationen erkennt.

Lagasse et al., "Purified hematopoietic stem cells can differenciate into hepatocytes in vivo", Nat Med, 11: 1229-1234 (2000), Kopen et al., "Marrow stromal cells migrate throughout forebrain and cerebellum, and they differenciate into astrocytes after injection into neonatal mouse brains", Procedings of the National Academy of Science USA, 96: 10711-10716 (1999), Brazelton et al., "From marrow to brain: expression of neuronal phenotypes in adult mice", Science 290: 1775-1779 (2000), zeigten, daß mesenchymale Stammzellen, die aus Knochenmark isoliert wurden, sich auch in nicht-mesenchymale Zellen wie Leberzellen, neuronale und gliale Zellen differenzieren konnten. Darüber hinaus konnte erst kürzlich gezeigt werden, daß mesenchymale Stammzellen aus adultem menschlichem. Knochenmark in neurale Zellen *in vitro* differenzieren konnten. Woodbury et al., "Adult rat and human bone marrow stromal cells differenciate into neurons", J. Neurosci. Res. 61: 364-370 (2000), zeigten, daß in Anwesenheit von Dimethylsulfoxid (DMSO) und β-Mercaptoethanol (BME) mesenchymale Stammzellen in Zellen differenziert werden konnten, die Neurofilament und Neuronen-spezifische Enolase exprimierten. Andere Forschergruppen beschrieben die Differenzierung von Stroma-Knochenmarkszellen mittels des epidermalen Wachstumsfaktors und dem "brain-derived neurotrophic factor" (BDNF) in Nervenzellen, die Nestin, das gliale fibrilläre saure Protein ("glial fibrillary acedic proteine", GFAP) und das Neuronen-spezifische nucleäre Protein (Neu-N) exprimierten.

Die Tatsache, daß mesenchymale Stammzellen unter bestimmten Bedingungen auch in Nervenzellen differenzieren können, bringt u.a. das Bedürfnis mit sich, diese mesenchymalen Stammzellen von neuronalen Vorläuferzellen unterscheiden zu können.

Diese neuronalen Vorläuferzellen (= Neural Progenitor Cells, im folgenden NPC) sind im zentralen Nervensystem zu finden. Sie exprimieren ebenfalls Nestin und können in Neuronen, Astrocyten und Oligodendrocyten differenzieren.

Neuronale Vorläuferzellen sind CD133- positiv; dieser Zelloberflächenmarker wurde ursprünglich auf hämatopoetischen Stammzellen gefunden. In letzter Zeit konnte jedoch gezeigt werden, daß dieser Marker auch von Nerven- und Skelettmuskelgewebe exprimiert wird. Aus diesen Gründen ist dieser Marker alleine für Unterscheidungszwecke verschiedener Stamm- oder Vorläuferzellen nicht geeignet.

Trotz des großen Interesses an mesenchymalen Stammzellen gibt es bis heute noch kein definiertes Protokoll für die Isolierung und Expansion der Zellen in Kultur. Die meisten Experimente zielten auf Kulturen von mesenchymalen Stammzellen ab, die insbesondere durch ihre feste Adharierung an Gewebekulturschalen isoliert werden konnten.

Da ferner mesenchymale Stammzellen und bspw. neuronale Vorläuferzellen als homogene Populationen angesehen werden, insbesondere bezüglich ihrer Morphologie und ihres Phänotyps, besteht auch aus diesen Gründen das Bedürfnis, zumindest diese beiden Stammzellen und Vorläuferzellen unterscheiden zu können.

Aus der US 5,837,539 sind Antikörper bekannt, die für Zelloberflächendeterminanten von menschlichen mesenchymalen Stammzellen spezifisch sind. Zur Generierung dieser Antikörper wurden Mäuse mit verschiedenen menschlichen mesenchymalen Stammzellen aus dem Knochenmark über mehrere Tage immunisiert.

Diese Antikörper wurden insbesondere dazu verwendet, um mesenchymale Stammzellen von hämatopoetischen Zellen zu unterscheiden. Darüber hinaus zeigte sich, daß diese Antikörper auch mit anderen nicht-mesenchymalen Zellen kreuzreagierten.

Vor diesem Hintergrund ist es Aufgabe der vorliegenden Erfindung, einen neuen monoklonalen Antikörper bereitzustellen, mit dem es möglich ist, in einer Stammzellen-enthaltenden Kultur mesenchymale Stammzellen zu identifizieren und ggf. zu trennen.

Erfindungsgemäß wird diese Aufgabe durch einen Antikörper oder ein Fragment davon gelöst, der an ein gleiches Antigen bindet wie ein Antikörper, der von der am 14.08.2002 bei der Deutschen Sammlung von Mikroorganismen und Zellkulturen (DSMZ) nach dem Budapester Vertrag unter der Nummer DSM ACC2567 hinterlegten Hybridomzellinie W8B2 produziert wird.

Die der Erfindung zugrundeliegende Aufgabe wird dadurch vollkommen gelöst.

Die Erfinder konnten in eigenen Versuchen zeigen, daß es mit dem neuen erfindungsgemäßen Antikörper möglich ist, selektiv mesenchymale Stammzellen zu isolieren und zu charakterisieren.

Den Erfindern ist es darüber hinaus gelungen, mit Hilfe des neuen Antikörpers mesenchymale Stammzellen bspw. von neuronalen Vorläuferzellen ausgezeichnet zu unterscheiden.

Ein derartiger Antikörper, mit dem es möglich ist, selektiv mesenchymale Stammzellen zu identifizieren und insbesondere von neuronalen Vorläuferzellen zu unterscheiden, ist nach Wissen der Erfinder bisher nicht erzeugt worden.

Die Erfinder konnten den Antikörper unter Verwendung der Zellinie WERI-RB-1 erzeugen.

Diese Zellinie ist eine aus einem Retinoblastom isolierte Zellinie, die z.B. bei der Deutschen Sammlung für Mikroorganismen und Zellkulturen unter der Nummer DSMZ ACC 90 geführt wird. Es war nicht zu erwarten - und im Fachgebiet fand sich auch kein Hinweis darauf -, daß unter Verwendung dieser Zellinie Antikörper zur Identifizierung von mesenchymalen Stammzellen generiert werden können.

Insofern war es überraschend, daß der Antikörper W8B2 an ein Antigen bindet, das für mesenchymale Stammzellen derart charakteristisch ist, daß er eine ausgezeichnete Möglichkeit bietet, mesenchymale Stammzellen aus einer verschiedene Zellpopulationen enthaltene Probe selektiv zu identifizieren. Die zur Immunisierung verwendete Zellinie ist bereits ausdifferenziert, weshalb nicht zu erwarten war, daß eine pluripotente mesenchymale Stammzelle aufgrund ihrer unterschiedlichen Oberflächenmarker von dem erfindungsgemäßen Antikörper erkannt werden würde.

Im Rahmen der vorliegenden Erfindung kann statt des jeweils angesprochenen Antikörpers auch ein Fragment des Antikörpers verwendet werden, ohne daß dies jeweils ausdrücklich erwähnt wird. Unter "Fragment" wird dabei jedes Fragment des Antikörpers verstanden, das die Antigenbindungsfunktion des Antikörpers beibehält. Solche Fragmente sind bspw. F_{ab}, F_{(ab},₎₂, Fᵥ und andere Fragmente wie z. B. CDR ("complementarity determining region", hypervariable Region) -Fragmente. Die genannten Fragmente weisen die Bindungsspezifität des Antikörpers auf und können auch bspw. mit bekannten Verfahren rekombinant hergestellt werden.

Die Produktion von monoklonalen Antikörpern durch die Fusion von Milzzellen von immunisierten Mäusen und Myelomzellen wurde bereits 1975 von Kohler und Milstein ("Continuous cultures of fused cells secreting antibody of predefined specificity", Nature 256: 495-497) beschrieben. Die Techniken für die chemische Selektion der Hybridome, die aus einer solchen Fusion resultieren, und die nachfolgende Isolation von Zellklonen, die einzelne Antikörper sekretieren, für die Herstellung von monoklonalen Antikörpern sind ebenso im Fachgebiet bekannt.

Die Erfindung betrifft insbesondere einen Antikörper oder ein Fragment davon, der von der Hybridomzellinie W8B2 produziert wird, die am 14.08.2002 bei der DSMZ nach dem Budapester Vertrag unter der Nummer DSM ACC2567 hinterlegt wurde.

Die Erfinder konnten in eigenen Versuchen zeigen, daß der obengenannte Antikörper spezifisch für mesenchymale Stammzellen ist. Die Erfinder konnten ferner in eigenen Versuchen zeigen, daß mesenchymale Stammzellen, die einen bekannten Immunophänotyp aufwiesen (jeweils positiv für CD10, CD13, CD61, CD90, CD105 (Endoglin)), mit dem neuen Antikörper in Subpopulationen fraktioniert werden konnten.

Ferner erwies sich der von der Hybridomzellinie W8B2 produzierte Antikörper vorteilhafterweise als nicht-reaktiv mit Knochenmark-mononucleären Zellen (BMMNC). Dadurch kann erreicht werden, daß die Selektion für mesenchymale Stammzellen äußerst spezifisch ist.

Der erfindungsgemäße Antikörper ermöglicht es jetzt auch, weitere Antikörper herzustellen, die an das gleiche Antigen binden. Durch den erfindungsgemäßen Antikörper ist es möglich, die entsprechende Antigenstruktur mit allgemein bekannten Verfahren zu isolieren und weitere monoklonale Antikörper gegen die gleiche Antigenstruktur zu entwickeln, wobei auch hier die bekannten Verfahren Anwendung finden.

Die Erfindung betrifft ferner eine Hybridomzellinie, die die Fähigkeit besitzt, derartige Antikörper zu produzieren und freizusetzen, und insbesondere die Hybridomzellinie W8B2.

Die Erfinder haben mit dem neuen Antikörper erstmals einen monoklonalen Antikörper sowie eine diesen produzierende und freisetzende Hybridomzellinie hergestellt, der eine gezielte Erkennung von mesenchymalen Stammzellen ermöglicht. Der Antikörper stellt somit ein bisher einzigartiges Mittel für den Arzt und Forscher dar, um einerseits derartige Zellen nachzuweisen und um andererseits diese Zellen ggf. zu manipulieren, entweder durch den Antikörper selbst oder durch daran gekoppelte Reagenzien.

Weiterhin betrifft die Erfindung ein Verfahren zur Isolierung und/oder Identifizierung von mesenchymalen Stammzellen, bei dem ein Antikörper oder ein Fragment davon verwendet wird, der an das gleiche Antigen bindet wie ein Antikörper, der von der am 14.08.2002 bei der DSMZ nach dem Budapester Vertrag unter der Nummer DSM ACC2567 hinterlegten Hybridomzellinie W8B2 produziert wird.

Dabei wird insbesondere ein Antikörper oder Fragmente davon verwendet, der von der am 14.08.2002 bei der DSMZ nach dem Budapester Vertrag unter der Nummer DSM ACC2567 hinterlegten Hybridomzellinie W8B2 produziert wird.

Die Erfinder haben erkannt, daß es mit dem erfindungsgemäßen Verfahren möglich ist, insbesondere mesenchymale Stammzellen zu identifizieren und bspw. gegen neuronale Vorläuferzellen abzugrenzen.

Die Erfindung betrifft insbesondere ein Verfahren zur Identifizierung von mesenchymalen Stammzellen mit einem Antikörper, das die folgenden Schritte umfaßt:
a) In-Verbindung-Bringen einer Probe, die mesenchymale Stammzellen enthält, mit den neuen monoklonalen Antikörper oder Fragmenten davon, und
b) Identifizieren solcher Zellen in der Probe, die die neuen monoklonalen Antikörper, oder Fragmente davon binden.

Weiterhin betrifft die Erfindung ein Verfahren zur Isolierung von mesenchymalen Stammzellen mit einem Antikörper, mit den folgenden Schritten:
(a) In-Verbindung-Bringen einer Probe einer zellsuspension, die mesenchymale Stammzellen enthält, mit den neuen monoklonalen Antikörper oder Fragmenten davon, und
(b) Isolieren solcher Zellen aus der Probe, die die neuen monoklonalen Antikörper, oder Fragmente davon binden.

Die Probe kann ausgewählt sein aus jeder Quelle, die mesenchymale Stammzellen enthält, also bspw. einer Probe aus dem Knochenmark, Blut oder Gewebe. Diese Zellen werden nach im Stand der Technik bekannten Labormethoden erhalten.

Das In-Verbindung-Bringen einer Probe einer Zellsuspension, die mesenchymale Stammzellen enthält, kann dabei in Lösung vollzogen werden, wie es bspw. bei Anwendung eines Durchflußzytometers (= fluorescent-activated cell sorter (FACS)) der Fall ist.

Bei der Durchflußzytometrie werden Zellen mit Antikörpern beladen, die einerseits spezifisch für einen Oberflächenmarker und andererseits mit einem Fluoreszenzfarbstoff gekoppelt sind. Diejenigen Zellen, die Marker-positiv sind, fluoreszieren, während die negativen Zellen dunkel bleiben. Es kann also festgestellt werden, welcher Anteil einer Zellpopulation Marker-positiv ist. Gleichzeitig erlaubt ein Durchflußzytometer, die Größe und Granularität von Zellen zu erfassen.

Eingesetzt werden kann auch ein Verfahren zur magnetischen Zellseparation (MACS, magnetic cell sorting). Bei diesem Verfahren werden die Zellen mit magnetischen Beads markiert, wobei diese Beads bspw. an die Antikörper gekoppelt sein können.

Ferner kann das In-Verbindung-Bringen auch dadurch durchgeführt werden, daß die monoklonalen Antikörper an einem Träger immobilisiert wird, wie es bspw. bei der Säulenchromatographie der Fall ist.

Nach Mischen der Zellsuspension mit den Antikörpern binden diejenigen Zellen die Antikörper, die das betreffende Antigen exprimieren, woraufhin diese Zellen von den Zellen, die keine Antikörper gebunden haben, nach dem beschriebenen Verfahren unterschieden und/oder isoliert werden können.

Die auf diese Weise isolierten und/oder identifizierten mesenchymalen Stammzellen können bspw. für eine Transplantation eingesetzt werden, wodurch eine Regenerierung von Knochen, Knorpel, Sehnen, Muskeln, Fettgewebe oder Stroma erreicht werden soll.

Die Erfindung betrifft weiterhin die Verwendung des neuen Antikörpers oder eines Fragmentes davon zur Isolierung und/oder Identifizierung von mesenchymalen Stammzellen.

Weiterhin betrifft die Erfindung die Verwendung der Zellinie WERI-RB-1 zur Herstellung von Antikörpern oder Fragmenten davon zur Isolierung und/oder Identifizierung von mesenchymalen Stammzellen.

Die Erfindung betrifft ferner eine pharmazeutische Zusammensetzung enthaltend den neuen erfindungsgemäßen Antikörper oder Fragmente davon.

Eine derartige pharmazeutische Zusammensetzung kann neben dem einen oder mehreren Antikörpern weitere geeignete Substanzen enthalten, wie bspw. Verdünnungsmittel, Lösungsmittel, Stabilisatoren usw. Hierzu zählen bspw. physiologische Kochsalzlösungen, Wasser, Alkohole, und weitere geeignete Substanzen, die bspw. in A. Kibble, "Handbook of Pharmaceutical Excipients", 3. Ed., 2000, American Pharmaceutical Association and Pharmaceutical Press, zu finden sind.

Die Erfindung betrifft ferner ein Kit, das den neuen Antikörper oder Fragmente davon enthält.

Weitere Vorteile ergeben sich aus den beigefügten Figuren und der Beschreibung.

Es versteht sich, daß die vorstehend genannten und die nachstehend noch zu erläuternden Merkmale nicht nur in der jeweils angegebenen Kombination, sondern auch in Alleinstellung oder in anderen Kombinationen verwendet werden können, ohne den Rahmen der vorliegenden Erfindung zu verlassen.

Ausführungsbeispiele sind in der beigefügten Zeichnung dargestellt und werden in der Beschreibung näher erläutert. Es zeigt:

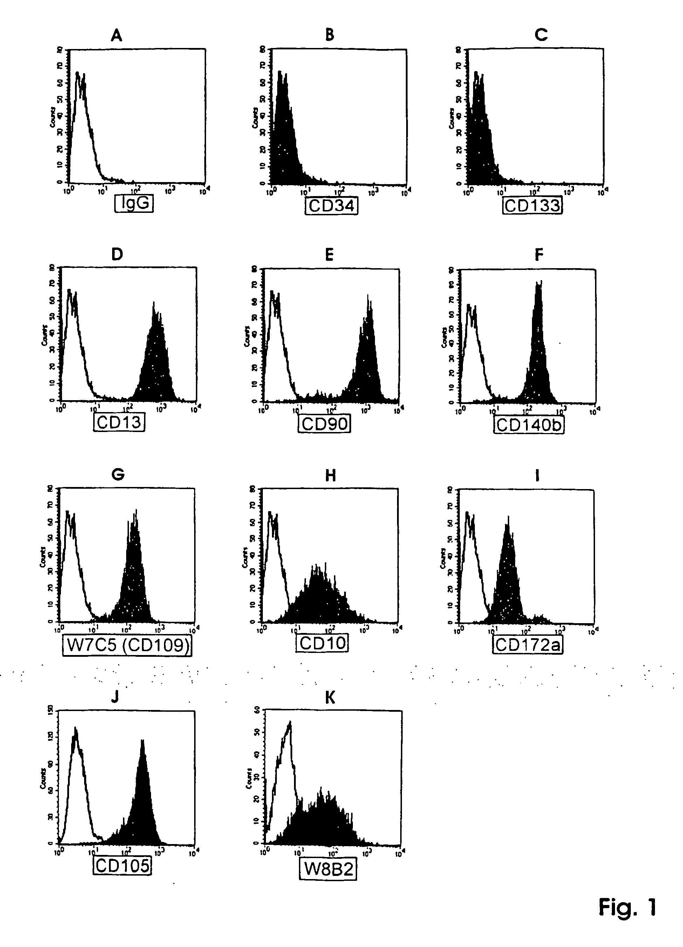

- Fig. 1: FACS-Analysen, die zeigen, daß MSC CD13, CD90, CD140B, W7C5 (CD109), CD10, CD172A, CD105 und den neuen Marker W8B2 exprimieren.

### Beispiel

### Material und Methoden

### Isolierung und Kulturen von MSC :

Mesenchymale Stammzellen wurden auf zwei verschiedene Arten erhalten: In einem Ansatz wurden sie von den Erfindern selber isoliert, in einem zweiten Ansatz wurden weitere mesenchymale Stammzellen bei der Firma CellSystems, St. Katharinen, Deutschland, käuflich erworben. Die mesenchymalen Stammzellen beider Ansätze wurden in anschließenden Studien miteinander verglichen.

Zur Gewinnung der mesenchymalen Stammzellen wurden Freiwilligen Spendern (n = 10) Knochenmarkszellen des Beckenraumes entnommen. Die Zellen des Knochenmarks wurden durch Dichtegradientenzentrifugation aufgetrennt und die mesenchymalen Stammzellen aus der Interphase isoliert (Biocoll-Trennlösung, 1,077 g/ml, Biochrom KG, Berlin, Deutschland).

Die getrennten Zellen wurden in RPMI 1640-Medium unter Beifügung von Glutamax, 1 % nicht-essentielle Aminosäuren, 1 % Natriumpyruvat (GIBCOBRL, Paisely, Schottland), 1 % Penicillin/Streptomycin (Biochrom KG) und 10 % fetalem Kälberserum (PAA Laboratories, Linz, Österreich) kultiviert. Die Zellen wurden mit 2,0 x 10⁵ Zellen/cm² in Gewebekulturflaschen (75 cm², Cellstar, Frickenhausen, Deutschland) gehalten und bei 37°C in einer befeuchteten Atmosphäre (5 % CO₂) inkubiert. Zwei Tage nach der Inkubation wurden die nicht-adhärierenden Zellen durch den Ersatz mit neuem Medium entfernt. Anschließend wurden die Zellen durch Inkubation mit 0,25 % Trypsin/l mM EDTA für 3 Minuten bei 37°C gelöst. Die mesenchymalen Stammzellen wurden fünf Mal passagiert und subkultiviert. Die erste Passage der MSC wurde für immunhistochemische Studien mit einer Dichte von 1,0 x 10⁴ Zellen/cm² auf 8-Well-Objektträgern kultiviert (Falcon, Heidelberg, Deutschland).

Die folgenden monoklonalen Antikörper oder Antikörper-Konjugate wurden für die fluorometrischen Analysen eingesetzt: W8B2, W8C3, W4A5 und W7C5, die allesamt ausgehend von der Retinoblastomzellinie WERI-RB-1 erhalten wurden. Diese Zellinie ist bei der DSMZ unter der Nummer ACC90 erhältlich.

Ferner eingesetzt wurden der monoklonale Antikörper 57D2, der durch die Immunisierung von Mäusen mit der TF-1 erythroleukämischen Zellinie (DSMZ: ACC334) erhalten wurde.

Als Antikörper mit bekannten Spezifitäten wurden CD10-PE, CD13-PE, CD34-PE, CD45-PE, CD56-PE, CD61-PE, CD117-PE eingesetzt (alle erhältlich bei Becton Dickinson, Heidelberg, Deutschland). PE (Phycoerythrin)-konjugierte monoklonale Antikörper mit einer Spezifität für CD90, CD140B und CD164 wurden von PharMingen (San Diego, USA) erworben. Von Sigma (München, Deutschland) wurde der Anti-Nervenwachstumfaktorrezeptor-Antikörper (NGFR) käuflich erworben. CD133-PE (Klon W6B3C1), CD167a (Kon 48B3), CD172a-PE (Klon SE5A5) und der CD105-spezifische monoklonale Antikörper 43A3 wurden in dem Labor der Erfinder hergestellt. Nicht-konjugierte Antikörper wurden mit Isotyp-spezifischen PE-konjugierten Ziege-Anti-Maus-Antiseren gefärbt (Southern Biotechnology Associates, Inc., Birmingham, USA).

### Anfärben der Zellen und Durchflußzytometrie

Für die zytometrischen Analysen wurden die Trypsin-behandelten MSC mit 10 µl Phycoerythrin-konjugierten Antikörpern oder 25 µl Kulturüberstand bei 4°C für 20 Minuten in 96-Well-Mikrotiterplatten inkubiert. Nicht-konjugierte monoklonale Antikörper wurden nach einem Waschschritt in FACS-Puffer (PBS; 0,5 % BSA; 0,1 % NaN₃) mit Ziege-Anti-Maus-IgG1-PE (1:100) oder Ziege-Anti-Maus-IgG3-PE (1:20) Antiseren gefärbt. Nach einem weiteren Waschschritt wurden die Zellen mit einem Durchflußzytometer (FACSCalibur, Becton Dickinson) unter Verwendung der Cell-Quest-Software (Bacton Dickinson) analysiert.

Für die immunzytochemische Analyse von intrazellulären Antigenen und extrazellulären Matrixproteinen wurden die mesenchymalen Stammzellen auf 8-Well-Kammerobjektträgern mit Aceton für 2 Minuten fixiert und mit dem primären Antikörper für 60 Minuten markiert. Anschließend wurde mit Alexa 488-konjugierten Ziegen-Anti-Maus-IgG- oder Ziegen-Anti-Kaninchen-IgG-Antiseren gefärbt. Für die Kontrollen wurden Zellen entweder mit einem Isotypen-passenden Kontrollantikörper oder mit einem Prä-Immun-Kaninchenserum markiert. Die Fluoreszenz der Zellen wurde mit einem Fluoreszenzmikroskop (Zeiss, Oberkochen, Deutschland) e-valuiert.

### RT-PCR-Analysen

Die Gesamt-RNA wurde aus Zell-Lysaten unter Verwendung des High-Pure-RNA-Isolation-Kit (Roche Molecular Biochemicals, Mannheim, Deutschland) nach Anleitung des Herstellers isoliert. Dieses Protokoll schließt eine Inkubation mit DNase mit ein, um kontaminierende DNA zu verdauen. Ungefähr 1 µg Gesamt-RNA wurde einer 20 µl cDNA-Synthesereaktion unterzogen, unter Verwendung der Zufallsprimer: 1^{st} Strand cDNA-Synthese-Kit für RT-PCR (AMV), Roche. 2 µl der cDNA wurden für die PCR-Amplifikation eingesetzt. Um die Integrität der RNA und die Effizienz der cDNA-Synthese zu kontrollieren, wurde 1 µl der cDNA mittels einem Intron-überspannenden Primerpaar für das β₂-Microglobulingen amplifiziert.

10 µl der PRC-Reaktion wurden auf einem 3%igen Agarosegel analysiert und mit Ethidiumbromid visualisiert.

### Ergebnisse

### Immunophänotyp von MSC

Der Phänotyp von kommerziell erhältlichem MSC (2 Passagen) wurde mit dem Phänotyp von MSC verglichen, die in dem Labor der Erfinder hergestellt wurden (s.o.). Bezüglich des letzten Falles wurde Knochenmarkszellen von gesunden Spendern in Gewebekulturflaschen (75 cm²) mit einer Dichte von 2,0 x 10⁵ Zellen/cm² über 10 bis 14 Tage in RPMI-Medium mit 10 % fetalem Kälberserum kultiviert. Die daraus resultierende adhärierende MSC-Population war heterogen und bestand aus Fibroblastenähnlichen Zellen zusätzlich zu runden und polygonalen Zellen unterschiedlicher Größe.

Der Phänotyp dieser MSC wurde bei verschiedenen Passagen analysiert (Passagen 1 - 5 = 14 - 61 Kulturtage).

Durchflußzytometrische Analysen zeigten, daß die MSC aller Passagen, einschließlich der kommerziell erhältlichen MSC, CD10, CD13, CD56, CD61, CD90, CD105 (Endoglin), CD140b (PDGF-RB), CD164 und CD172a (SIRPα) und auch die Antigene, die durch die erfindungsgemäß hergestellten Antikörper W8B2 und W7C5 (CD109) definiert werden, exprimierten. Sie waren jedoch negativ für CD15, CD45, CD34, CD117, CD133 und CD167a.

In Fig. 1 sind ausgewählte Beispiele dieser Analysen dargestellt. So ist in den Histogrammen B und C der Fig. 1 zu sehen, daß die mesenchymalen Stammzellen eindeutig negativ für die Marker CD34 und CD133 sind. In den meisten Fällen waren die Expressionsmuster der kommerziell erhältlichen MSC und der MSC, die in dem Labor der Erfinder hergestellt wurden, identisch oder zumindest ähnlich. In denHistogrammen G und K der Fig. 1 ist zu erkennen, daß die mesenchymalen Stammzellen W7C5 (CD109) und W8B2 exprimieren.

Die Ergebnisse der Untersuchungen der Erfinder sind in der folgenden Tabelle 1 zusammengefaßt, in der die Antigen-Expression auf mesenchymalen Stammzellen mit der auf mononucleären Zellen aus dem Knochenmark von gesunden Spendern verglichen wird.

In der Tabelle bedeuten - negativ, d.h. keine Expression auf den betreffenden Zellen, + positiv, d.h. eine Expression auf den Zellen, (+) zumindest in einer Analyse positiv, S geringe bis nicht detektierbare Expression, P Zellpopulation < 5 %.

| Antigen/ Antikörper | MSC | MSC | BMMNC |
|---|---|---|---|
| | lab. | Komm. | |
| CD13 | + | + | P |
| CD34 | - | - | p |
| CD45 | - | - | + |
| CD56 | S | S | P |
| CD90 | + | + | P |
| CD105 | + | + | P |
| CD117 | - | - | P |
| CD133 | - | - | P |
| CD140B | + | + | P |
| CD164 | + | + | + |
| CD167 | - | S | - |
| CD172a | + | + | + |
| W4A5 | - | - | - |
| W6D3 (CD15) | - | - | + |
| W7C5 (CD109) | + | + | P |
| W8B2 | + | - | - |
| NGFR | (+) | (+) | - |

Die Erfinder konnten darüber hinaus zeigen, daß die Antigen-Expression auf MSC in Abhängigkeit der Anzahl der Passagen heterogen war. Mit Bezug auf die W7C5 (CD109) Antigen-Expression konnte eine schrittweise Abnahme auf 10 % des ursprünglichen Werts mit Bezug auf die erste bis zur fünften Passage gemessen werden.

Darüber hinaus sank die Reaktivität des monoklonalen Antikörpers W8B2 mit MSC mit zunehmender Anzahl der Passage bedeutend.

Daher konnte eine Status-spezifische Expression und die Heterogenität unter den MSC-Populationen gezeigt werden.

In weiteren Studien wurde untersucht, in wie weit die für mesenchymale Stammzellen spezifischen Antikörper W8B2 und W7C5 (CD109) bspw. an neuronale Vorläuferzellen binden. Hierzu wurden neuronale Vorläuferzellen eingesetzt, die bei der Firma CellSystems (St. Katharinen, Deutschland) käuflich erworben wurden. Die neuronalen Vorläuferzellen erwiesen sich als eindeutig negativ für den neuen Antikörper W8B2 und den Antikörper W7C5 (CD109). Demnach ist der erfindungsgemäße Antikörper ein ausgezeichnetes Mittel, mit dem bspw. zwischen mesenchymalen Stammzellen und neuronalen Vorläuferzellen unterschieden werden kann.

Um die Expression von intrazellulären Differenzierungs-Antigenen und extrazellulären Matrixproteinen zu analysieren, wurde eine Immunfluoreszenzanalyse von MSC durchgeführt, die auf Kammer-Objektträgern wuchsen. Als Negativkontrolle wurden ungefärbte Zellen verwendet. In den meisten MSC wurde eine starke intrazelluläre Expression von Vimentin festgestellt, jedoch nur eine geringe und heterogene Expression von Nestin in kleinen einzelnen Zellen, ebenso wie in größeren Zellen mit polygonalem Erscheinungsbild. Darüber hinaus exprimierten die MSC-Kulturen Fibronectin. Cytoplasmatisches Neu-N, β2-Ketten-Laminin und die neuronalen Marker NF, GFAP, β-Tubulin und MAP-2 waren negativ.

### m-RNA-Expression in MSC

Unter Einsatz der Reversen Transkriptase-PCR (RT-PCR) wurde die Expression von Nestin, MAP-2, Neurofilamente, GFAP und β2-Ketten-Laminin mRNA analysiert. In Übereinstimmung mit den immunophänotypischen Analysen konnte Nestin und Vimentin mRNA detektiert werden. Darüber hinaus konnte auch eine β2-Ketten-Laminin mRNA-Expression beobachtet werden. Wie erwartet konnten keine Neurofilamente, GFAP oder MAP-2 mRNA in mesenchymalen Stammzellen gefunden werden.

### Zusammenfassung

Die von den Erfindern durchgeführten Studien zeigten, daß MSC typischerweise für die folgenden Antigene negativ waren: CD45, CD34, CD133. Die MSC waren positiv für CD10, CD13, CD61, CD90, CD105 (Endoglin). Dieser Immunophänotyp bleibt über mehrere Kulturpassagen hinweg konsistent und ist mit anderen veröffentlichten Daten in Übereinstimmung. Die Erfinder konnten darüber hinaus zum ersten Mal zeigen, daß MSC auch CD140b (PDGF-RB), CD164 und CD172a (SIRPα) exprimieren. Ferner war der neue Antikörper W8B2, der nicht mit BMMNC reagiert, dazu in der Lage, die MSC-Kulturen in Subpopulationen zu fraktionieren. Darüber hinaus konnte gezeigt werden, daß der Stammzell-Antikörper W7C5 (CD109) ein Antigen definiert, dessen Intensität von der Anzahl der Passagen der MSC-Kulturen abhängt. MSC verlieren bekanntermaßen nach der Kultur von Primärzellen die Oberflächenantigene SH3, ICAM-1 und Integrinβ1 und senken ihre Produktion an extrazellulären Matrixmolekülen. Im Gegensatz dazu wurde das Antigen W8B2 nach Kultur der primären Knochenmarkszellen in den MSC der ersten Passage hochreguliert und anschließend nach mehreren weiteren Passagen herunterreguliert. Diese Moleküle können aus diesen Gründen eine wichtige Rolle bezüglich des Proliferations- oder Differenzierungspotentials von MSC spielen. Darüber hinaus waren MSC auch für Vimentin und Fibronectin positiv.

## Patentansprüche

1. Monoklonaler Antikörper oder Fragment davon zur Isolierung und/oder Identifizierung von mesenchymalen Stammzellen, **dadurch gekennzeichnet, daß** der Antikörper oder das Fragment davon an ein gleiches Antigen bindet wie ein Antikörper, der von der am 14.08.2002 bei der Deutschen Sammlung von Mikroorganismen und Zellkulturen (DSMZ) nach dem Budapester Vertrag unter der Nummer DSM ACC2567 hinterlegten Hybridomzellinie W8B2 produziert wird.

2. Monoklonaler Antikörper oder Fragment davon zur Identifizierung und/oder Isolierung von mesenchymalen Seammzellen, **dadurch gekennzeichnet, daß** er von der Hybridomzellinie W8B2 produziert wird, die am 14.08.2002 bei der DSMZ nach dem Budapester Vertrag unter der Nummer DSM ACC2567 hinterlegt wurde.

3. Hybridomzellinie, die einen Antikörper nach Anspruch 1 oder 2 produziert.

4. Verfahren zur in-vitro-Isolierung und/oder Identifizierung von mesenchymalen Stammzellen mit einem Antikörper, **dadurch gekennzeichnet, daß** ein Antikörper oder ein Fragment davon verwendet wird, der an das gleiche Antigen bindet wie ein Antikörper, der von der am 14.08.2002 bei der DSMZ nach dem Budapester Vertrag unter der Nummer DSM ACC2567 hinterlegten Hybridomzellinie W8B2 produziert wird.

5. Verfahren zur *in-vitro*-Isolierung und/oder Identifizierung von mesenchymalen Stammzellen mit einem Antikörper, **dadurch gekennzeichnet, daß** ein Antikörper oder ein Fragment davon verwendet wird, der von der am 14.08.2002 bei der DSMZ nach dem Budapester Vertrag unter der Nummer DSM ACC2567 hinterlegten Hybridomzellinie W8B2 produziert wird.

6. Verfahren zur Isolierung und/oder Identifizierung von mesenchymalen Stammzellen mit einem Antikörper, mit den folgenden Schritten:
(a) In-Verbindung-Bringen einer Probe einer Zellsuspension, die mesenchymale Stammzellen enthält, mit einem monoklonalen Antikörper nach Anspruch 1 oder 2 oder einem Fragment davon, und
(b) Identifizieren solcher Zellen in der Probe, die die monoklonalen Antikörper oder Fragmente davon binden.

7. Verfahren zur Isolierung von mesenchymalen Stammzellen mit einem Antikörper, mit den folgenden Schritten:
(a) In-verbindung-Bringen einer Probe einer Zellsuspension, die mesenchymale Stammzellen enthält, mit einem monoklonalen Antikörper nach Anspruch 1 oder 2 oder Fragmenten davon, und
(b) Isolieren solcher Zellen aus der Probe, die die monoklonalen Antikörper oder Fragmente davon binden.

8. Verwendung eines Antikörpers oder eines Fragments davon nach Anspruch 1 oder 2 zur in-vitro-Isolierung und/oder Identifizierung von mesenchymalen Stammzellen.

9. Verwendung der bei der DSMZ unter der Nummer ACC90 hinterlegten Zellinie WERI-RB-1 zur Herstellung von Antikörpern oder Fragmenten davon zur in-vitro-Isolierung und/oder Identifizierung von mesenchymalen Stammzellen.

10. Pharmazeutische Zusammensetzung enthaltend einen Antikörper oder Fragmente davon nach Anspruch 1 oder 2.

11. Kit enthaltend einen Antikörper oder Fragmente davon nach Anspruch 1 oder 2.

## Claims

1. Monoclonal antibody, or fragment thereof, for isolating and/or identifying mesenchymal stem cells, **characterized in that** the antibody, or the fragment thereof, binds to the same antigen as an antibody which is produced by the hybridoma cell line W8B2, which was deposited, in accordance with the Budapest Treaty, on 14.08.2002 in the Deutsche Sammlung von Mikroorganismen und Zellkulturen (DSMZ) under the number DSM ACC2567.

2. Monoclonal antibody, or fragment thereof, for identifying and/or isolating mesenchymal stem cells, **characterized in that** it is produced by the hybridoma cell line W8B2, which was deposited, in accordance with the Budapest Treaty, on 14.08.2002 in the DSMZ under the number DSM ACC2567.

3. Hybridoma cell line which produces an antibody according to claim 1 or 2.

4. Method for isolating and/or identifying mesenchymal stem cells *in vitro,* using an antibody, **characterized in that** an antibody, or a fragment thereof is used, which binds to the same antigen as an antibody which is produced by the hybridoma cell line W8B2, which was deposited, in accordance with the Budapest Treaty, on 14.08.2002 in the DSMZ under the number DSM ACC2567.

5. Method for isolating and/or identifying mesenchymal stem cells *in vitro,* using an antibody, **characterized in that** an antibody, or a fragment thereof, is used which is produced by the hybridoma cell line W8B2, which was deposited, in accordance with the Budapest Treaty, on 14.08.2002 in the DSMZ under the number DSM ACC2567.

6. Method for isolating and/or identifying mesenchymal stem cells using an antibody, comprising the following steps:
a) contacting a sample of a cell suspension containing mesenchymal stem cells to a monoclonal antibody according to claim 1 or 2, or a fragment thereof, and
b) identifying the cells in the sample which bind the monoclonal antibodies, or fragments thereof.

7. Method for isolating mesenchymal stem cells using an antibody, comprising the following steps:
(a) contacting a sample of a cell suspension containing mesenchymal stem cells to a monoclonal antibody according to claim 1 or 2, or fragments thereof, and
(b) isolating the cells in the sample which bind the monoclonal antibodies, or fragments thereof.

8. Use of an antibody, or a fragment thereof, according to claim 1 or 2 for isolating and/or identifying mesenchymal stem cells *in vitro.*

9. Use of the cell line WERI-RB-1, which is deposited in the DSMZ under the number ACC90, for preparing antibodies, or fragments thereof, for isolating and/or identifying mesenchymal stem cells *in vitro.*

10. Pharmaceutical composition comprising an antibody, or fragments thereof, according to claim 1 or 2.

11. Kit containing an antibody, or fragments thereof, according to claim 1 or 2.

## Revendications

1. Anticorps monoclonal ou fragment de celui-ci pour l'isolement et/ou l'identification de cellules souches mésenchymateuses, **caractérisé en ce que** l'anticorps ou son fragment lie un même antigène qu'un anticorps qui est produit par la lignée cellulaire d'hybridome W8B2, déposée !e 14.08.2002 au Deutschen Sammlung von Mikroorganismen und Zellkulturen (DSMZ) selon le Traité de Budapest, sous le numéro DSM ACC2567.

2. Anticorps monoclonal ou fragment de celui-ci pour l'identification et/ou l'isolement de cellules souches mésenchymateuses, **caractérisé en ce qu'**il est produit par la lignée cellulaire d'hybridome W8B2, qui a été déposée le 14.08.2002 au DSMZ selon le Traité de Budapest, sous le numéro DSM ACC2567.

3. Lignée cellulaire d'hybridome, qui produit un anticorps selon la revendication 1 ou 2.

4. Procédé d'isolement *in vitro* et/ou d'identification de cellules souches mésenchymateuses avec un anticorps, **caractérisé en ce que** l'on utilise un anticorps ou son fragment, qui lie le même antigène qu'un anticorps qui est produit par la lignée cellulaire d'hybridome W8B2, déposée le 14.08.2002 au DSMZ selon le Traité de Budapest, sous le numéro DSM ACC2567.

5. Procédé d'isolement *in vitro* et/ou d'identification de cellules souches mésenchymateuses avec un anticorps, **caractérisé en ce que** l'on utilise un anticorps ou son fragment, qui est produit par la lignée cellulaire d'hybridome W8B2, déposée le 14.08.2002 au DSMZ selon le Traité de Budapest, sous le numéro DSM ACC2567.

6. Procédé d'isolement et/ou d'identification de cellules souches mésenchymateuses avec un anticorps, comprenant les étapes suivantes :
(a) mettre en contact un échantillon d'une suspension qui contient des cellules souches mésenchymateuses, avec un anticorps monoclonal selon la revendication 1 ou 2 ou son fragment, et
(b) identifier les cellules de l'échantillon, qui se lient à l'anticorps monoclonal ou à son fragment.

7. Procédé d'isolement de cellules souches mésenchymateuses avec un anticorps, comprenant les étapes suivantes :
(a) mettre en contact un échantillon d'une suspension qui contient des cellules souches mésenchymateuses, avec un anticorps monoclonal selon la revendication 1 ou 2 ou son fragment, et
(b) isoler les cellules de l'échantillon, qui se lient à l'anticorps monoclonal ou à son fragment.

8. Utilisation d'un anticorps ou de son fragment, selon la revendication 1 ou 2, pour l'isolement *in vitro* et/ou l'identification de cellules souches mésenchymateuses.

9. Utilisation de la lignée cellulaire WERI-RB-1, déposée au DSMZ sous le numéro ACC90, pour la préparation d'anticorps ou de fragments de ceux-ci pour l'isolement *in vitro* et/ou l'identification de cellules souches mésenchymateuses.

10. Composition pharmaceutique contenant un anticorps ou fragment de celui-ci selon la revendication 1 ou 2.

11. Kit contenant un anticorps ou fragment de celui-ci selon la revendication 1 ou 2
